# EUROPÄISCHE PATENTANMELDUNG

(11) **EP 2 181 721 A2**
(43) Veröffentlichungstag der Anmeldung: **05.05.2010**
(21) Anmeldenummer: 10000324.3
(22) Anmeldetag: 06.11.2006
(51) Int. Cl.: A61L 15/32, A61K 9/00

(54) **Produkt zur Versorgung von Entzündungen, Druckstellen und/oder Aphten im Oralbereich sowie Verwendung eines solchen Produkts**

(62) Teilanmeldung aus: 06023033.1
(71) Anmelder: Lohmann & Rauscher GmbH & Co. KG, 56567 Neuwied (DE)
(72) Erfinder: Scholz, Gert Hartmut Dr., 56567 Neuwied (DE)
(74) Vertreter: Kraus, Jürgen Helmut

(57) **Zusammenfassung**

Die Erfindung betrifft ein Produkt zur Versorgung von Entzündungen, Druckstellen und/oder Aphthen im Oralbereich aus einem überwiegend, vorzugsweise vollständig, bioresorbierbaren Material, wobei das bioresorbierbare Material ein an der Schleimhaut haftendes, flexibles und/oder kompressibles Polsterelement aufweist, insbes. vollständig als Polsterelement ausgeführt ist.

## Beschreibung

Die Erfindung betrifft ein Produkt zur Versorgung von Entzündungen, Druckstellen und/oder Aphthen im Oralbereich aus einem überwiegend, vorzugsweise vollständig, bioresorbierbaren Material sowie die Verwendung eines derartigen Produktes.

Beschwerden im Oralbereich können unterschiedliche Ursachen haben. Beispielsweise ist die Mundschleimhaut dann anfälliger, wenn sie zu trocken ist. Das kommt manchmal mit steigendem Alter vor, kann aber auch als Nebenwirkung von bestimmten Medikamenten auftreten. Auch bei einem geschwächten Immunsystem können Entzündungen der Mundschleimhaut auftreten. Eine andere Ursache für Probleme im Oralbereich können Vitaminmangel oder allergische Reaktionen sein. Dabei können häufige Entzündungen der Mundschleimhaut auf einen Mangel an bestimmten Vitaminen oder Eisen hindeuten. Auch allergische Reaktionen auf Speisen oder Medikamente können die Ursache sein.

Besonders häufig werden Probleme im Oralbereich durch Zahnprothesen verursacht. Dabei gibt es im besonderen bei der Neuanpassung von Teil- oder Vollprothesen im Ober- und Unterkieferbereich oft lange Eingewöhnungszeiten von mehreren Wochen, während denen Schmerzen und Druckstellen auftreten können. Ähnliche Probleme treten bei Zahnspangen auf. Diese können an der Mundschleimhaut regelrecht scheuern und dadurch Druckstellen und Entzündungen verursachen. Auch bei zahnärztlichen Eingriffen sind Druckstellen durch Instrumente manchmal kaum zu vermeiden. Oft entstehen nach einer Behandlung an solchen Stellen erst nach Stunden oder wenigen Tagen kleine Wunden. Verletzungen im Mundraum können auch durch den Einsatz von Zahnstochern, Gabeln, Knochen, Gräten oder aber durch einen abgebrochenen, scharfkantigen Zahn auftreten. Es kommt ebenfalls nicht selten vor, daß man sich versehentlich auf die Wangen, die Lippen oder die Zunge beißt. Manchmal ist es sogar eine zu harte Zahnbürste, die beim Zähneputzen das Zahnfleisch oft zunächst erst unbemerkt verletzt. Ferner gibt es Mundschleimhautentzündungen, deren Ursache nicht eindeutig feststellbar ist. Ursache davon können beispielsweise Pilzinfektionen (Mundsoor), Virusinfektionen (z.B. mit Herpesviren) oder durch Bakterien verursachte Infektionen sein.

Eine der häufigsten Krankheiten der Mundschleimhaut sind die sog. Aphthen. Aphthen sind meist linsengroße, schmerzhafte Schleimhautveränderungen, die von einem roten entzündlichen Hof umgeben sind. Am häufigsten treten sie am Mundboden, an der Wangen- und Zungenschleimhaut und an der Innenseite der Lippen auf. Manchmal werden sie durch Verletzungen ausgelöst, wie sie oben beschrieben wurden, oder sie treten einfach als unangenehme Begleiterscheinung von Infektionskrankheiten auf. Die eigentliche Ursache konnte die Wissenschaft jedoch bis heute nicht feststellen.

Zur Behandlung der gerade beschriebenen Entzündungen, Druckstellen und/oder Aphthen im Oralbereich sind zahlreiche Präparate bekannt. Diese Präparate liegen in Form von Lösungen zum Spülen, Gelen, Sprays, Pastillen oder Lutschtabletten vor. Sie enthalten Wirkstoffe wie Benzalkonium oder Cetrimonium, Chlorhexidin, Cetylpyridiniumchlorid, Cresol, Dichlorbenzylalkohol oder Hexetidin. Der Wirkstoff Tyrothricin ist ein Antibiotikum, das die entzündungsauslösenden Bakterien unschädlich macht. Ferner sind einige pflanzliche Mittel bekannt, die desinfizierend wirken und Entzündungen hemmen. Auch hier gibt es zahlreiche Darreichungsformen, z.B. Salben, Gele, Sprays, Lösungen und Tinkturen. Sie enthalten beispielsweise Auszüge von Heilpflanzen wie Salbei, Kamille, Pfefferminze, Echinacea (Sonnenhut), Nelkenbaum, Myrrhenstrauch und Rhabarber. Pfefferminz- und Nelkenöl zeigen neben antiseptischen auch örtlich betäubende Effekte. Echinaceaextrakte aktivieren das körpereigene Abwehrsystem. Myrrhetinkturen und Rhabarberwurzelextrakte wirken adstringierend und schützen dadurch die Schleimhäute.

Mit den bekannten Präparaten zur Versorgung von Entzündungen, Druckstellen und/oder Aphthen werden beachtliche Erfolge erzielt. Diese Präparate müssen allerdings oft in hoher Dosierung verwendet werden. Ferner kommt es in vielen Fällen, insbes. bei Problemen im Bereich von Prothesen oder Zahnspangen, bisweilen kurz nach Absetzung der Präparate zu erneuten Entzündungen und/oder Druckstellen.

Angesichts der genannten Probleme im Stand der Technik liegt der Erfindung die Aufgabe zugrunde, Produkte zur Versorgung von Entzündungen, Druckstellen und/oder Aphthen bereitzustellen, mit denen bei geringer Dosierung nachhaltig Heilungserfolge erzielt werden können.

Erfindungsgemäß wird diese Aufgabe durch eine Weiterbildung der bekannten Produkte gelöst, die im wesentlichen dadurch gekennzeichnet ist, daß das bioresorbierbare Material ein an der Schleimhaut haftendes, flexibles und/oder kompressibles Polsterelement aufweist, insbes. vollständig als Polsterelement ausgeführt ist.

Diese Erfindung geht auf die Erkenntnis zurück, daß die Probleme im Stand der Technik hinsichtlich der zum Erhalt des gewünschten Heilungserfolgs erforderlichen hohen Dosierung im wesentlichen darauf zurückgehen, daß sich die üblicherweise eingesetzten Lösungen, Gele oder Sprays im gesamten Mundraum verteilen, so daß zur Behandlung lokaler Entzündungen oder Druckstellen große Mengen entsprechender Mittel benutzt werden müssen. Dieses Problem kann beseitigt werden, wenn das zur Versorgung von Entzündungen, Druckstellen und/oder Aphthen eingesetzte bioresorbierbare Material ein an der Schleimhaut haftendes Polsterelement aufweist. Das bioresorbierbare Material kann dann gezielt an die problematische Stelle im Mundraum gebracht werden. Eine übermäßig hohe Wirkstoffmenge ist bei Einsatz erfindungsgemäßer Produkte nicht mehr erforderlich.

Gemäß einem weiteren Gesichtspunkt der Erfindung ist das bioresorbierbare Material als flexibles und/oder kompressibles Polsterelement ausgeführt. Dieser Gesichtspunkt der Erfindung geht auf die Erkenntnis zurück, daß das rasche Wiederauftreten von Problemen im Bereich von Vollprothesen und/oder Zahnspangen durch den von diesen Elementen ausgeübten lokalen Druck verursacht wird. Dieser lokale Druck kann mit Hilfe erfindungsgemäß ausgeführter flexibler und/oder kompressibler Polsterelemente über größere Bereiche verteilt bzw. gedämpft werden. Dadurch kann die Ursache für Druckstellen und Entzündungen mit Hilfe erfindungsgemäßer Produkte beseitigt werden.

Im Hinblick auf die gewünschte Bioresorbierbarkeit bei gleichzeitiger Sicherstellung flexibler und/oder kompressibler Eigenschaften hat es sich als zweckmäßig erwiesen, wenn das bioresorbierbare Material ein vorzugsweise mit mindestens einem Begleitstoff versetztes Kollagen aufweist. Kollagen kann in Form von kompressiblen Schwämmen bereitgestellt werden, die bei Befeuchten an der Mundschleimhaut haften, im trockenen Zustand aber keine haftenden Eigenschaften aufweisen. Das macht die Lagerung entsprechender Produkte einfach und sichert gleichzeitig die erfindungsgemäß gewünschten Wirkungen hinsichtlich der Haftungseigenschaften an der Schleimhaut, der Flexibilität und/oder der Kompressibilität. Verfahren zum Herstellen von zur Herstellung erfindungsgemäßer Produkte einsetzbaren Kollagenschäumen sind beispielsweise in der DE 38 32 162 C2 beschrieben. Der Offenbarungsgehalt dieser Schrift hinsichtlich der Herstellung von Kollagenschäumen wird hiermit durch ausdrückliche Inbezugnahme in diese Beschreibung aufgenommen. Dabei kann es sich bei dem erfindungsgemäß einsetzbaren Kollagen um marktübliches bovines, equines, porkines und/oder marines Kollagen handeln. Zum Erhalt gewünschter Eigenschaften, wie etwa zum Erhalt einer gewünschten Flexibilität und Kompressibilität kann das bioresorbierbare Material Kollagen und mindestens ein weiteres bioresorbierbares Polymer aufweisen. Dabei kann das weitere bioresorbierbare Polymer ein Alginat, Gellan, Chitosan, Chitin, κ-Karagenan, ϕ-Karagenan, Xanthan, Carboxymethylcellulose, Hydroxyethylcellulose und/oder Hydroxyethylpropylcellulose aufweisen.

Unter diesen Stoffen beeinflußt ein Zusatz von Alginat die Freisetzung von polykationischen Wirkstoffen (z.B. Biguanide wie Polyhexanid). Dabei kann von einer kontinuierlichen Wirkstoffabgabe ausgegangen werden. Das Ca-Alginat beeinflußt die mechanische Belastbarkeit und Biodegradierbarkeit. Ferner ist bei Einsatz von Ca-Alginat ein positiver Nutzen in der Wundheilung beschrieben. Besonders hervorzuheben ist, daß durch die Zugabe von Ca-Alginat zu Kollagen eine besonders hohe reversible Kompressibilität des bioresorbierbaren Materials erreicht werden kann.

Gellan kann die Freisetzung von Biguaniden ebenfalls beeinflussen. Dazu ist in der Literatur im Hinblick auf Gellan ebenfalls eine wundheilungsfördernde Wirkung beschrieben.

Chitosan besitzt einen bakteriostatischen Effekt. Außerdem besitzt Chitosan einen hämostatischen Effekt. Dieser wirkt sich auch auf die Kombination mit der bioresorbierbaren Matrix aus. Es kommt zu einer Erhöhung der hämostatischen und der bakteriostatischen Wirkung. Ferner stellt Chitosan einen schlechten Nährboden für Mikroorganismen dar. Daher verhindert der Einsatz von Chitosan ein schnelles Verkeimen der Kollagenauflage, die von Natur aus einen guten Nährboden für Mikroorganismen darstellt. In einer besonderen Ausführungsform wird Chitosan in einer löslichen Form zugegeben, so daß es aus der Kollagenmatrix austritt und durch seine filmbildenden Eigenschaften das Mikroklima im Mundraum positiv beeinflußt. Chitin ist in Wasser nicht löslich. Daher wird Chitin zweckmäßigerweise in Form eines Copolymers von Chitosan und Chitin eingesetzt. Dieses Copolymer hat etwa die gleichen Eigenschaften wie Chitosan.

In der Kombination mit Biguaniden, wie z.B. Polyhexanid, kommt es durch eine Veränderung der ionischen Ladung der Kollagenmatrix zu einer erhöhten Freisetzung des Wirkstoffs.

κ-Karagenan ist biodegradierbar und besitzt nur eine geringe Löslichkeit. Daher kann durch Zugabe von κ-Karagenan die Kollagenmatrix hydrophober eingestellt werden.

ϕ-Karagenan besitzt eine bessere Löslichkeit. Es fungiert insbes. in der durchspeichelten Probe zur Verbesserung des Mundgefühls.

Xanthan ist ein sehr effektives Verdickungsmittel. Dies führt ebenfalls zu einer Verbesserung des Mundgefühls, insbes. in einer durchspeichelten Kollagenmatrix.

Die Zugabe von Carboxymethylcellulose führt ebenfalls zu einer Verbesserung des Mundgefühls. Aufgrund der polyanionischen Natur der Komponente wird die Abgabe von Biguaniden herabgesetzt. Es wird ein Verkeimen der Auflage verhindert. Die Wirkstoffabgabe (z.B. Polyhexanid) ist äußerst gering.

Hydroxyethylcellulose und Hydroxyethylpropylcellulose führen in Kombination mit Kollagen zu einer Verbesserung des Mundgefühls.

Die Konzentration des weiteren bioresorbierbaren Materials beträgt vorzugsweise 0,5 bis 50 %, bezogen auf das Trockengewicht des bioresorbierbaren Trägermaterials (Kollagen). Bei einer Konzentration von weniger als 0,5 % werden die angestrebten Wirkungen nicht erhalten. Bei einer Konzentration von 50 % oder mehr werden die Eigenschaften des Trägermaterials nachteilhaft beeinflußt. Beispielsweise besteht die Gefahr, daß die gewünschte Polsterwirkung dann nicht mehr erreicht wird. Das gilt allerdings nicht für Honig. Dieser kann auch in höheren Konzentrationen zugesetzt werden, ohne die Eigenschaften des Trägermaterials negativ zu beeinflussen.

Gemäß einer weiteren Ausführungsform der Erfindung weist das bioresorbierbare Material mindestens einen die Erhöhung der Haftung an der Schleimhaut, die Verbesserung der Flexibilität bzw. Anschmiegsamkeit und/oder die Verbesserung des Geschmacks bewirkenden Begleitstoff auf. Dieser Begleitstoff kann Honig, Propolis, Aloe Vera, Panthenol, Polyethylenglykol 200-2000, Polyvinylpyrrolidon, Glycerin, Kamilleextrakt, Salbeiöl, Pfefferminzöl, Vitamine und/oder Provitamine aufweisen. Honig kann in einer Konzentration von 60 % oder mehr, bezogen auf den Trockengehalt der Kollagenmatrix, zugesetzt werden. Glycerin kann in einer Menge von 0,1 bis 50 %, bezogen auf den Trockengehalt der Kollagenmatrix, zugesetzt werden. Der Kamilleextrakt wird vorzugsweise in einer Menge von 0,5 bis 1,5 %, insbes. etwa 1 %, bezogen auf den Trockengehalt der Kollagenmatrix, hinzugesetzt. Ebenso wird das Salbeiöl vorzugsweise in einer Menge von 0,5 bis 1,5 %, insbes. etwa 1 %, bezogen auf den Trockengehalt der Kollagenmatrix, hinzugesetzt. Vitamin C kann ebenfalls in einer Menge von 0,5 bis 1,5 %, bezogen auf den Trockengehalt der Kollagenmatrix, insbes. in einer Menge von etwa 1 %, beigefügt werden. Zitronenextrakt wird ebenfalls in einer Menge von 0,5 bis 1,5 %, bezogen auf den Trockengehalt der Kollagenmatrix, insbes. in einer Menge von etwa 1 %, beigefügt.

Die bislang genannten Begleitstoffe Honig, Glycerin, Kamilleextrakt, Salbeiöl, Pfefferminzöl und Vitamin C sowie Zitronenextrakt, Cyclamat und andere synthetische Süßstoffe dienen ebenso wie der ebenfalls als Begleitstoff einsetzbare Orangenextrakt zur Geschmacksverbesserung. Dabei werden Cyclamat und andere synthetische Süßstoffe vorzugsweise in einer Menge von 0,2 bis 0,8 %, insbes. etwa 0,5 %, bezogen auf den Trokkengehalt der Kollagenmatrix, eingesetzt, während Orangenextrakt in einer Menge von 0,5 bis 1,5 %, insbes. etwa 1 %, bezogen auf den Trockengehalt der Kollagenmatrix, beigefügt wird. Minzöle, insbes. Pfefferminze (*Mentha x piperita*), wird zweckmäßigerweise in einer Menge von 0,5 bis 1,5 % eingesetzt.

Eine Geruchsverbesserung kann durch Zugabe von Propolis in einer Menge von 0,5 bis 1,5 %, insbes. etwa 1 %, bezogen auf den Trockengehalt der Kollagenmatrix ebenso erzielt werden wie durch Zugabe von Extrakten von Zitrusfrüchten in einer Menge von 0,5 bis 1,5 %, insbes. etwa 1 %, bezogen auf den Trockengehalt der Kollagenmatrix.

Eine haftungsverändernde Wirkung wird durch Glycerin, Panthenol, Polyethylenglykol 200-2000, Polyvinylpyrrolidon, Aloe Vera oder auch durch andere biodegradierbare Polymere erzielt. Dabei beträgt die Glycerinmenge vorzugsweise etwa 5 bis 50 %, bezogen auf den Trockengehalt der Kollagenmatrix. Panthenol wird zweckmäßigerweise in einer Menge von 2 bis 5 %, bezogen auf den Trockengehalt der Kollagenmatrix, beigefügt. Polyethylenglykol 200-2000 kann in einer Menge von 1 bis 5 %, insbes. etwa 3 %, bezogen auf den Trockengehalt der Kollagenmatrix, eingesetzt werden. Polyvinylpyrrolidon wird zweckmäßigerweise in einer Menge von 1,5 bis 4,5 %, insbes. etwa 3 %, bezogen auf den Trokkengehalt der Kollagenmatrix, eingesetzt. Die Aloe Vera Menge beträgt vorzugsweise etwa 1 bis 3 %, insbes. etwa 2 %, bezogen auf den Trockengehalt der Kollagenmatrix.

Insgesamt ist es zweckmäßig, wenn die Konzentration der Begleitstoffe im Bereich von 0,5 bis 50 %, bezogen auf das Trockengewicht des Trägermaterials (Kollagen), liegt. Bei einer Konzentration von weniger als 0,5 % werden die gewünschten Wirkungen üblicherweise nicht erreicht, während bei einer Konzentration von mehr als 50 % die Eigenschaften der Kollagenmatrix nachteilhaft beeinflußt werden können.

Bei einer weiteren Ausführungsform der Erfindung kann der zum Versetzen des Kollagens benutzte Begleitstoff ein Anästhetikum und/oder Antiseptikum aufweisen, wobei das Antiseptikum bzw. Lokalanästhetikum aus der Gruppe der Ester bzw. Säureamide der Paraaminobenzoesäure ausgewählt sein kann. Dem Antiseptikum bzw. Lokalanästhetikum kann eine geringe Menge Adrenalin zur Wirkstoffverbesserung zugesetzt sein. Zweckmäßigerweise weist das Anästhetikum Ultracain, Lidocain, Mepivacain, Bupivacain, Prilocain, Articain, Procain und/oder Tetracain auf. Dabei beträgt die Menge des Ultracains, Lidocains und/oder Mepivacains vorzugsweise etwa 0,1 bis 0,3 %, insbes. etwa 0,2 %, bezogen auf den Trockengehalt der Kollagenmatrix. Bupivacain wird zweckmäßigerweise in einer Menge von 0,25 bis 0,75 %, insbes. etwa 0,5 %, bezogen auf den Trockengehalt der Kollagenmatrix, zugesetzt. Gleiches gilt für Prilocain. Articain, Procain und Tetracain gelten mittlerweile als veraltet und werden nur noch bei allergischen Reaktionen gegen den Amintyp verwendet. Dabei beträgt die Menge zweckmäßigerweise 0,5 bis 1 %, bezogen auf den Trockengehalt der Kollagenmatrix.

Üblicherweise werden Lokalanästhetika intramuskulär verabreicht oder oberflächlich in Form eines Gels auf das Zahnfleisch bzw. die Mundschleimhaut aufgetragen. Dabei ist es nachteilhaft, daß sich das Gel insbes. bei der Behandlung von Aphthen, die meist an der Unterlippe auftreten, schnell und stark im Mundraum verteilt. Hierdurch kommt es zu einer unangenehmen, weiträumigen Betäubung im Mundbereich. Dieses Problem kann durch den Einsatz einer biodegradierbaren Kollagenmatrix als Trägermaterial für das Lokalanästhetikum gelöst werden. Dabei wird das Lokalanästhetikum kontinuierlich an die Mundschleimhaut abgegeben. Es kommt somit nur zu einer lokalen Betäubung der Stelle, die auch wirklich betäubt werden soll. Das als Träger benutzte bioresorbierbare Material, wie etwa Kollagen, löst sich nicht so schnell auf wie ein ansonsten eingesetztes Gel, so daß mit vergleichsweise geringer Wirkstoffdosierung über einen längeren Zeitraum eine gute Betäubung des zu behandelnden Areals erreicht werden kann.

Ferner ist als weiterer Vorteil der Verwendung eines Lokalanästhetikums hervorzuheben, daß der Einsatz einer Spritze beim Zahnarzt den meisten Kindern, aber auch Erwachsenen, Angst macht. Ein Lokalanästhetikum, das zuvor an der Einstichstelle angewendet wird, bewirkt die Unterdrückung des Penetrationsschmerzes. Hierdurch verbinden Kinder diesen Moment nicht mehr mit einem schlechten Erlebnis und es kommt nicht zu einer Traumatisierung. Lokalanästhetika sind zwar verfügbar, sollen aber häufig nicht bei Kindern unter 12 Jahren verwendet werden. Außerdem schmecken sie nicht besonders gut. Eine Kollagenauflage gibt nur einen Bruchteil der Menge des Lokalanästhetikums ab, insbes. wenn es unter kontrollierten Bedingungen von einem Zahnarzt eingesetzt wird. Hierdurch kann eine solche Kollagenmatrix auch bei Kindern zum Einsatz kommen. Außerdem ist davon auszugehen, daß ein kleines Stück Kollagenmatrix mit einem Lokalanästhetikum, das beispielsweise süß schmeckt, von Kindern besser akzeptiert wird als eine Salbe oder ein Gel.

Die Konzentration des Antiseptikums bzw. Lokalanästhetikums liegt zweckmäßigerweise im Rahmen der physiologischen Wirksamkeit im Bereich von 0,2 bis 2 %, bezogen auf das Trockengewicht des Trägermaterials. Gemäß einem weiteren Gesichtspunkt der Erfindung kann das erfindungsgemäße Produkt einen eine antimikrobielle Verbindung aufweisenden Begleitstoff enthalten. Die antimikrobielle Verbindung kann aus dem Bereich der Biguanide stammen. Dabei kann es sich im besonderen um Polyhexamethylenbiguanid Hydrochlorid, Chlorhexidin Dihydrochlorid, Chlorhexidin Diacetat, Chlorhexidin D-Glukonat, Octenidin, Taurolidin, Cetylpyridiniumhydrochlorid und/oder Mischungen daraus handeln.

Häufig sind Wunden im Mundbereich infiziert bzw. es ist eine mikrobiologische Besiedlung der Wunde ursächlich für die Entstehung der Wunde. Als Beispiel dafür ist eine Traumatisierung bzw. eine Druckstelle zu nennen, die sich jederzeit in eine infizierte Wunde verwandeln kann. Weitaus häufiger ist im Dentalbereich jedoch Soor oder das Auftreten von Aphthen. Hierbei handelt es sich um eine Wundinfektion.

Wie eingangs bereits erläutert, besteht ein Problem der bekannterweise eingesetzten Präparate zur Behandlung dieser Probleme in der örtlichen Gabe von antiseptischen Substanzen. Ein Gel verteilt sich schnell im Mundraum und führt zu einer negativen Beeinflussung des Mikroklimas im Mundraum. Durch die Verteilung wird schnell eine zu geringe Wirkstoffkonzentration an der zu behandelnden Stelle erreicht. Dies führt zu einer unzureichenden bzw. keiner antimikrobiellen Wirksamkeit des Antiseptikums. Gleiches gilt für Gurgellösungen oder Gele, die bei einer Parodontosebehandlung zum Einsatz kommen. So wird bei einer Parodontosebehandlung ein antimikrobiell wirkendes Gel in eine passende Gebißform gegeben. Diese muß zuvor jedoch hergestellt worden sein. Dies generiert für die Kostenträger besonders hohe Kosten. Der Effekt, der hierdurch erreicht wird, ist eine antimikrobielle Wirkung über einen längeren Zeitraum, da sich das Gel durch die Gebißform nicht verteilen kann.

Eine Lösung für die genannten Verteilungs- bzw. Kostenprobleme stellt die erfindungsgemäß einsetzbare biodegradierbare Kollagenmatrix dar. Mit einer solchen Matrix wird an der infizierten Wunde kontinuierlich Wirkstoff freigesetzt. Aufgrund der festen Struktur des Trägermaterials bzw. der Kollagenmatrix kann es zu keiner Verteilung der Auflage kommen. Daher kann eine Wundinfektion für die Mundflora schonender und, durch eine längere Verweilzeit des Produktes, effektiver angegangen werden.

Sehr wirksame antimikrobielle Substanzen mit einer Breitbandwirkung gegenüber einer Vielzahl von Mikroorganismen stellen die Biguanide dar. Diese wirken auch gut gegenüber multiresistenten Keimen. Biguanide weisen nicht nur eine gute antibakterielle Wirkung auf, sondern auch eine sehr geringe Zytotoxizität. Dabei wird Polyhexanit als Begleitstoff zweckmäßigerweise in einer Menge von 0,5 bis 1 %, bezogen auf den Trockengehalt der Kollagenmatrix, eingesetzt. Chlorhexidin wird zweckmäßigerweise in einer Menge von 0,5 bis 1,5 %, insbes. etwa 1 %, bezogen auf den Trockengehalt der Kollagenmatrix, eingesetzt. Gleiches gilt für andere Chlorhexidine. Octenidin, Taurolidin und Cetylpyridiniumhydrochlorid werden ebenfalls zweckmäßigerweise in einer Menge von 0,5 bis 1,5 %, insbes. in einer Menge von etwa 1 %, bezogen auf den Trockengehalt der Kollagenmatrix, eingesetzt.

Die Konzentration der als Begleitstoff eingesetzten antimikrobiellen Verbindungen beträgt erfindungsgemäß im Rahmen der antimikrobiellen Wirksamkeit insgesamt zweckmäßigerweise 0,5 bis 2 %, bezogen auf das Trockengewicht des Trägermaterials.

Die antimikrobiellen Verbindungen können auch silberhaltige Verbindungen aufweisen. Dabei kann es sich um ein lösliches Silbersalz, ein schwerlösliches Silbersalz, Silberkomplexe, organische Silberverbindungen und/oder elementares Silber, vorzugsweise um Silberchlorid, handeln. Diese silberhaltigen Verbindungen können als Silbernitrat, Silberacetat, Silberkarbonat, Silberhalogenid, Silbersulfatiacin, Silberdiamin-Komplexe, Silberdithionat-Komplexe, Silberthiocyanat, Silberpulver und/oder Mischungen daraus vorliegen.

Bei der Behandlung infizierter Wunden zeigen Silberionen eine gute antimikrobielle Wirkung. Schon in geringer Konzentration (1 x 10⁻⁶ bis 1 x 10⁻⁹ Mol/l) sind Silberionen sehr wirksam zur Bekämpfung von Bakterien, Viren und Parasiten. Dabei werden häufig Silberprodukte mit einer geringen Löslichkeit eingesetzt, die dadurch bedingt nur geringe Mengen Silberionen freisetzen. Dazu gehört Silberchlorid, Silberbromid, Silberjodid, Silberoxid, Silberkarbonat, Silberthiocyanat und auch Silberpulver. Diese Substanzen geben nach ihrer Applikation kontinuierlich geringe Mengen Silber aus der Auflage frei. Die Silberionen werden bei ihrer antimikrobiellen Wirkung zu elementarem Silber reduziert. Hierdurch sinkt schnell die Konzentration der Silberionen in der Wundflüssigkeit. Da nur Silberionen eine antimikrobielle Wirkung aufweisen, ist es wesentlich, daß über einen längeren Zeitraum Silberionen in ausreichender Konzentration zur Verfügung stehen. Durch den Einsatz von schwer löslichen Silbersalzen werden gemäß dem chemischen Gleichgewicht kontinuierlich Silberionen nachgeliefert, solange noch Silbersalze in der Wundauflage vorhanden sind.

Eine infizierte Wunde benötigt am Anfang der Behandlung eine größere Menge Silber und im späteren Verlauf, nachdem die Keimzahl reduziert wurde, eine kontinuierliche Silberionenfreigabe. Dies kann erfindungsgemäß durch eine Mischung von Silber-Komplexen und schwerlöslichen Silberverbindungen in der polymeren Matrix erreicht werden. Hierbei werden zunächst die Silberkomplexe aus der Kollagenmatrix herausgelöst. Dadurch kommt es zu einer Stoßfreisetzung von Silber in der Wundflüssigkeit. Ein Zusatz an schwerlöslichen Silbersalzen sorgt für eine kontinuierliche Freigabe von Silber-Ionen über einen langen Zeitraum. Bei einem Ausführungsbeispiel werden in 1000 ml einer 1 % Kollagenmatrixlösung (Kollagen und weitere Hilfs- bzw. Zusatzstoffe) 5 ml einer Silberacetatlösung gegeben. Die Lösung enthält 15,46 mg Silberacetat. Nachdem die Lösung homogenisiert wurde, wurden 5 ml einer Natriumchloridlösung hinzugefügt. Die Lösung enthielt 5,4 mg Natriumchlorid. Alternativ kann auch erst die Silberacetatlösung mit der Natriumchloridlösung gemischt werden, die anschließend der Kollagendispersion zugegeben wird. Eine Zugabe von 29,85 mg Silberdithionat (Ag[S₂O₃]₂) ist im Anschluß möglich. Silberkonzentration: 0,1 % AgCl und 0,1 % Silber-Komplex.

Die Konzentration der silberhaltigen Verbindungen liegt im Rahmen der antimikrobiellen Wirksamkeit erfindungsgemäß zweckmäßigerweise im Bereich von 0,1 bis 2 %, bezogen auf das Trockengewicht des Trägermaterials.

Wie eingangs bereits erläutert, weist das bioresorbierbare Material zweckmäßigerweise einen feinporigen Schwamm auf, mit dem eine besonders gute Polsterwirkung erhalten werden kann. Durch die Porenstruktur nimmt die vorzugsweise als bioresorbierbares Material eingesetzte Kollagenmatrix viel Flüssigkeit auf. Ferner wird durch die schaumartige Struktur eine gute Kompressibilität erreicht. Der Schaum füllt daher besonders gut unebene oder zerklüftete Stellen aus. Ein trockener Kollagenschwamm haftet spontan am Zahnfleisch bzw. den Zähnen. Es hat sich empirisch gezeigt, daß ein filmartiges Material bei der Anwendung im Oralbereich deutlich schlechter haftet.

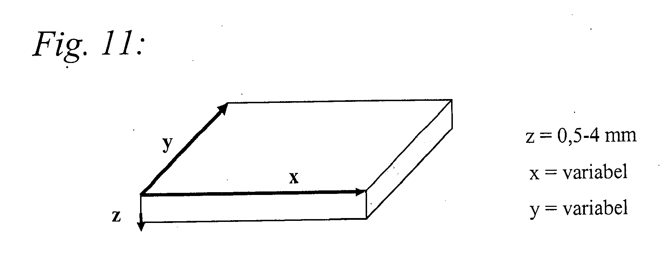

Zum Erhalt einer zufriedenstellenden Polsterwirkung bei gleichzeitiger Vermeidung einer unangenehmen Füllwirkung hat es sich als zweckmäßig erwiesen, wenn das bioresorbierbare Material einen Schaum bzw. Schwamm aufweist, der in Z-Richtung (vgl. Fig. 11) eine Dicke von 0,5 bis 4 mm aufweist.

Zweckmäßigerweise wird der Schaum in Sonderformen für den Dentalbereich geformt.

Als besonders vorteilhaft hat es sich erwiesen, wenn das erfindungsgemäße Produkt durch Strahlung (25 kGy) oder durch Begasen mit Ethylenoxid sterilisierbar ist.

Im Hinblick auf die angestrebte Polsterwirkung des erfindungsgemäßen Produktes hat es sich als zweckmäßig erwiesen, wenn das bioresorbierbare Material durch eine kurze Druckeinwirkung von 1 bar in Z-Richtung (vgl. Fig. 11) auf weniger als 50 %, vorzugsweise weniger als 40 %, insbes. 25 % oder weniger, der ursprünglichen Dicke komprimierbar ist.

Zusätzlich oder alternativ kann das bioresorbierbare Material im Rahmen der Erfindung, vorzugsweise bedingt durch einen Lyophilisationsprozeß, auch noch eine gute Verformbarkeit in X- und Y-Richtung (vgl. Fig. 11) aufweisen.

Im Rahmen der Verträglichkeit erfindungsgemäßer Produkte ist es angestrebt, daß das bioresorbierbare Material im Dentalbereich abbaubar ist und bei einem versehentlichen Verschlucken vollständig resorbiert wird. Zur Erhöhung der Haltbarkeit kann die Biodegradierbarkeit des bioresorbierbaren Materials durch zumindest teilweise Vernetzung verringert werden. Als zweckmäßig hat es sich erwiesen, wenn das bioresorbierbare Material mit bi- bzw. multifunktionellen Aldehyden, Carbonsäuren, Carbonsäurechloriden, Carbonsäurealdehyden und/oder Epoxiden vernetzbar ist. Zusätzlich oder alternativ kann die Biodegradierbarkeit des bioresorbierbaren Materials zweckmäßigerweise mit Hilfe spaltbarer Brükken sowie durch γ-Bestrahlung oder durch β-Bestrahlung verändert werden.

Wie der vorstehenden Erläuterung erfindungsgemäßer Produkte zu entnehmen ist, wird das bioresorbierbare Material im Rahmen der Erfindung zur Wundheilung und/oder Desinfektion (z.B. Behandlung von Aphthen, Druckstellen, Mykosen, Soor und anderen Infektionen) eingesetzt. Dazu kann das bioresorbierbare Material als Polster zwischen Zahnersatz bzw. Zahnspangen und der Mundschleimhaut, einer Wunde oder dem Zahnfleisch eingesetzt werden. Zusätzlich oder alternativ kann das bioresorbierbare Material auch als Haftmittel für Voll- oder Teilprothesen eingesetzt werden, wobei es ggf. durch Chitosan-Zusatz zur Hämostase bei Blutungen im Dentalbereich (auch bei Personen die unter dem Einfluß von Blutgerinnungshemmern, wie z.B. Marcumar, stehen) eingesetzt wird.

Vorstehend wurde bereits darauf hingewiesen, daß das erfindungsgemäß eingesetzte bioresorbierbare Material eine gute Haftung an der Mundschleimhaut aufweist. Das gilt im besonderen für bioresorbierbares Material in Form von Kollagen. Wenn eine kollagenhaltige Auflage im Mundraum plaziert wird, klebt diese sehr gut an Zähnen und Zahnfleisch. Zur quantitativen Bestimmung der Haftung wurden folgende Untersuchungen durchgeführt:

Ein Objektträger wurde mit einer 2,5 % warmen Agar-Lösung beschichtet. Die Agar-Lösung wurde komplett eingetrocknet. Die zu untersuchenden Proben wurden mit Speichel befeuchtet und auf einen beschichteten Objektträger gegeben. Ein zweiter beschichteter Objektträger wurde nach dem Bespeicheln der Probe an die Probe herangefahren (Abstand: 9,6 mm). Es wurde ein Kontakt von mind. einer Minute aufrechterhalten, bevor eine Zugmessung durchgeführt wurde. Die Probe weist eine Fläche von 15 mm x 20 mm auf. Die zur Ablösung der Probe von dem Objektträger erforderliche Zugkraft (Haftung) ist in der folgenden Tabelle dargestellt:

| Bezeichnung | F max [N] |
|---|---|
| Subrasorb C (Referenz) | 4,3 |
| Kollagen + 50 % Glycerin | 2,3 |
| Kollagen + 20 % PEG | 1,78; 1,54; 1,65 |
| Kollagen + 10 % Na-Alginat | 5,2 |
| Kollagen + 10 % Gellan | 5,0 |
| Kollagen + 10 % Polyarylsäure | 2,3 |
| Kollagen + 60 % Honig | 7,2 |
| Kollagen + 10 % Na- + 10 % Ca-Alginat | 4,5 |
| Kollagen + 10 % CMC | 1,8 |
| Kollagen + 60 % Honig + 5 % Propolis + 10 % Na-Alginat | 3,7 |

Demnach weist das erfindungsgemäße Produkt bzw. das bioresorbierbare Material des erfindungsgemäßen Produktes zweckmäßigerweise ein Haftungsvermögen von 1 Newton oder mehr, insbes. 2 Newton oder mehr, besonders bevorzugt 4 Newton oder mehr, auf.

Ferner wurden Untersuchungen zur Bestimmung der Kompressibilität unterschiedlicher Kollagenauflagen durchgeführt. Dazu wurden die Kollagenauflagen (bioresorbierbares Material) für 10 Sekunden mit einem Druck von 1 bar belastet. Es wurde die Dicke vor und nach der Belastung bestimmt. Außerdem wurde die Probe zwischen Zeigefinger und Daumen für einige Sekunden fest zusammengedrückt, um die maximale Kompressibilität einer trockenen Auflage zu bestimmen. Die Ergebnisse sind in der folgenden Tabelle dargestellt:

| Bezeichnung | Dicke unbelastet | Dicke 10 Sek. 1 bar | Dicke 10 Sek. > 5 bar | Bemerkung |
|---|---|---|---|---|
| Subrasorb C (Referenz) | 7,1 mm | 1,7 mm | 0,35 mm | reversibel |
| Kollagen + 30 % Glycerin | 6,9 | 0,6 | 0,24 | irreversibel |
| Kollagen + 50 % Glycerin | 5,7 | 0,38 | 0,26 | irreversibel |
| Kollagen + 10 % Propolis | 6,9 | 1,1 | 0,48 | irreversibel |
| Kollagen + 10 % CMC | 6,0 | 1,1 | 0,31 | irreversibel |
| Kollagen + 60 % Honig | 6,0 | 0,85 | 0,29 | irreversibel |
| Kollagen + 10 % Polyacrylsäure | 5,0 | 0,9 | 0,2 | irreversibel |
| Kollagen + 10 % Gellan | 7,0 | 0,9 | 0,34 | irreversibel |
| Kollagen + 10 % Na-Alginat | 6,0 | 0,8 | 0,27 | irreversibel |
| Kollagen + 10 % Na-Alginat + 60 % Honig + 5 % Propolis | 6,1 | 0,33 | 0,20 | irreversibel |
| Kollagen + 20 % PEG | 6,0 | 1,5 | 0,31 | reversibel |
| Kollagen + 10 % Na- + 10 % Ca-Alginat | 7,0 | 1,2 | 0,45 | reversibel |

Bei dem in der Tabelle angegebenen Referenzprodukt "Subrasorb C" handelt es sich um ein Produkt, das gemäß DE 38 32 162 C2 hergestellt wurde.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, näher erläutert. In der Zeichnung zeigt:
- **Fig. 1A**: ein erfindungsgemäßes Produkt für die Anwendung im Zusammenhang mit Vollprothesen im Unterkiefer,
- **Fig. 1B**: ein erfindungsgemäßes Produkt für die Anwendung im Zusammenhang mit Vollprothesen im Oberkiefer,
- **Fig. 2**: ein erfindungsgemäßes Produkt für die Versorgung von Zahnfleisch,
- **Fig. 3A und 3B**: einen z.B. zum Fixieren an einem Zahn einsetzbaren Kollagenschwamm mit einem Band,
- **Fig. 4**: einen eingeschnittenen Kollagenschwamm zum Abreißen,
- **Fig. 5**: einen zur Herstellung erfindungsgemäßer Produkte einsetzbaren Kollagenstreifen,
- **Fig. 6**: ein erfindungsgemäßes Produkt in Form eines U-förmigen Kollagenschwämmchens zur Versorgung eines Zahns,
- **Fig. 7**: einen Spender zur Bereitstellung erfindungsgemäßer Produkte,
- **Fig. 8**: ein erfindungsgemäßes Produkt zur Versorgung des Zahnfleisches über einigen Zähnen,
- **Fig. 9**: ein erfindungsgemäßes Produkt zur Anwendung im Zusammenhang mit Vollprothesen im Oberkiefer gemäß einer weiteren Ausführungsform der Erfindung,
- **Fig. 10**: ein variabel einsetzbares erfindungsgemäßes Produkt in ovaler Form und
- **Fig. 11**: eine 3-D-Darstellung des erfindungsgemäß einsetzbaren biodegradierbaren Materials.

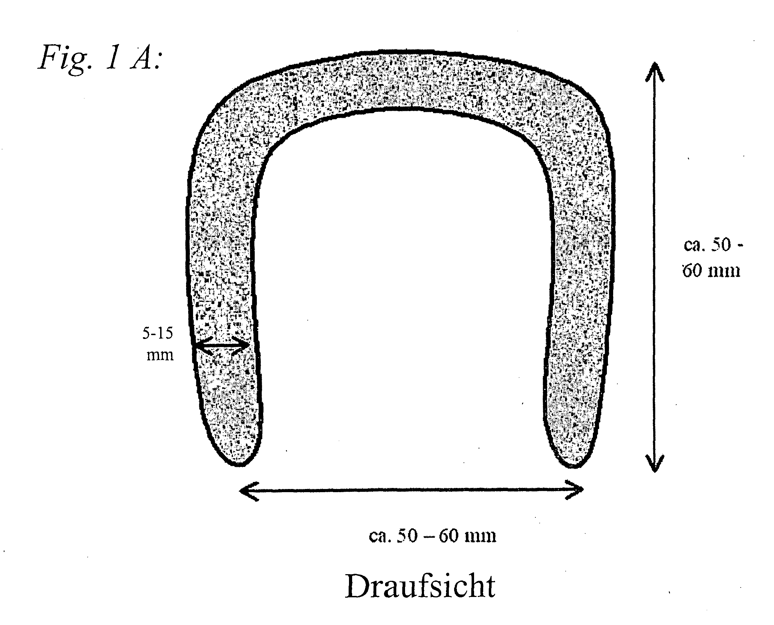

Das in Fig. 1A dargestellte Produkt zum Einsatz im Zusammenhang mit Vollprothesen im Unterkiefer ist im wesentlichen U-förmig ausgeführt. Es weist eine Breite zwischen den Schenkeln von etwa 50 bis 60 mm auf und eine Höhe von ebenfalls etwa 50 bis 60 mm. Die Materialstärke der Schenkel beträgt etwa 5 bis 15 mm.

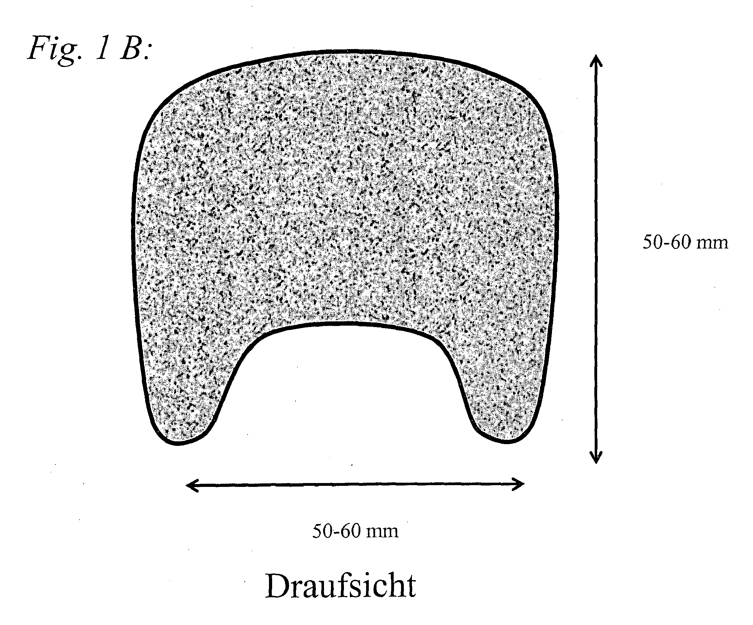

Das in Fig. 1B dargestellte Produkt entspricht hinsichtlich der äußeren Form im wesentlichen dem in Fig. 1A dargestellten Produkt. Es ist jedoch zwischen den Schenkeln ausgefüllt, so daß es den Raum zwischen einer Vollprothese im Oberkiefer und dem Gaumen nahezu vollständig ausfüllen kann.

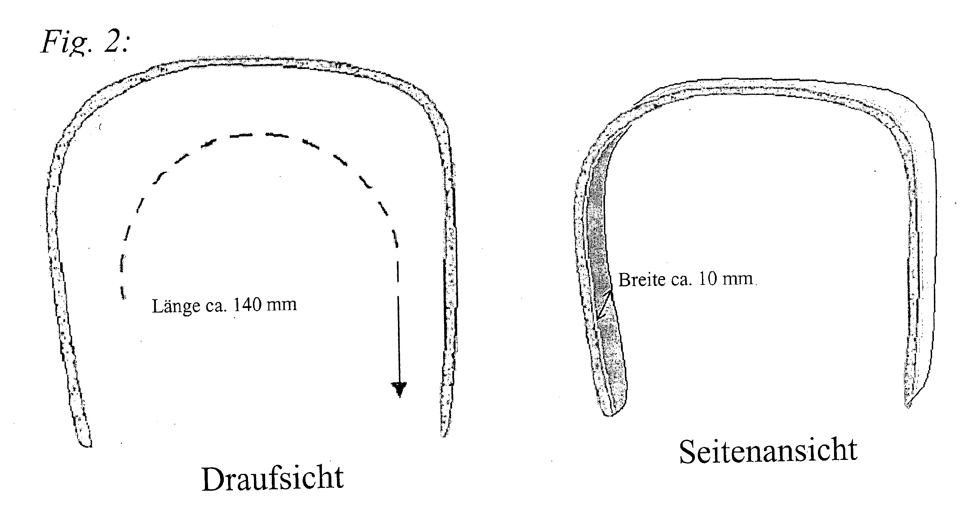

Das in Fig. 2 dargestellte erfindungsgemäße Produkt zur Versorgung von Zahnfleisch ist ebenfalls im wesentlichen U-förmig ausgeführt. Es weist eine Länge von etwa 140 mm auf, wobei die Materialstärke gemäß der Seitenansicht etwa 10 mm beträgt.

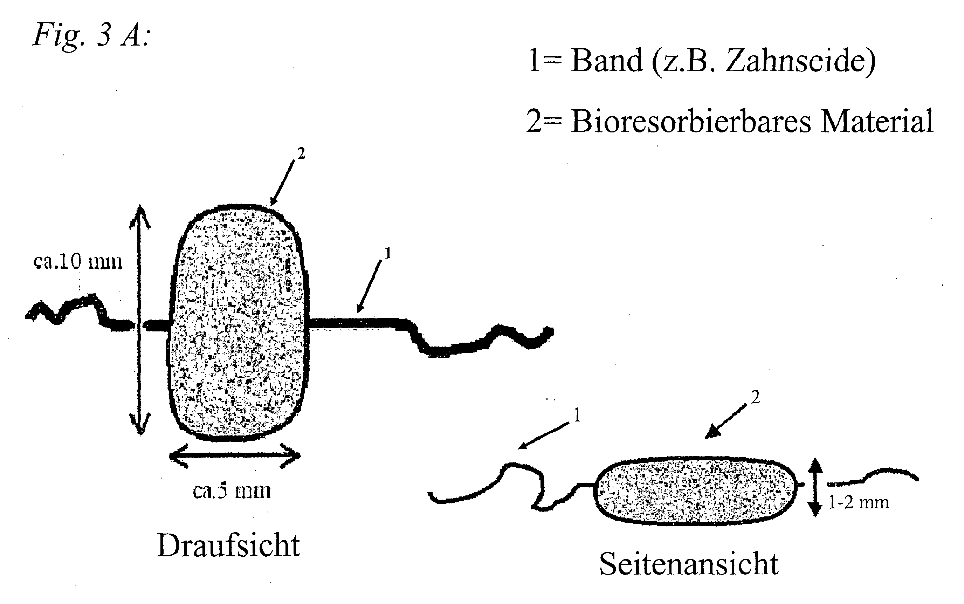

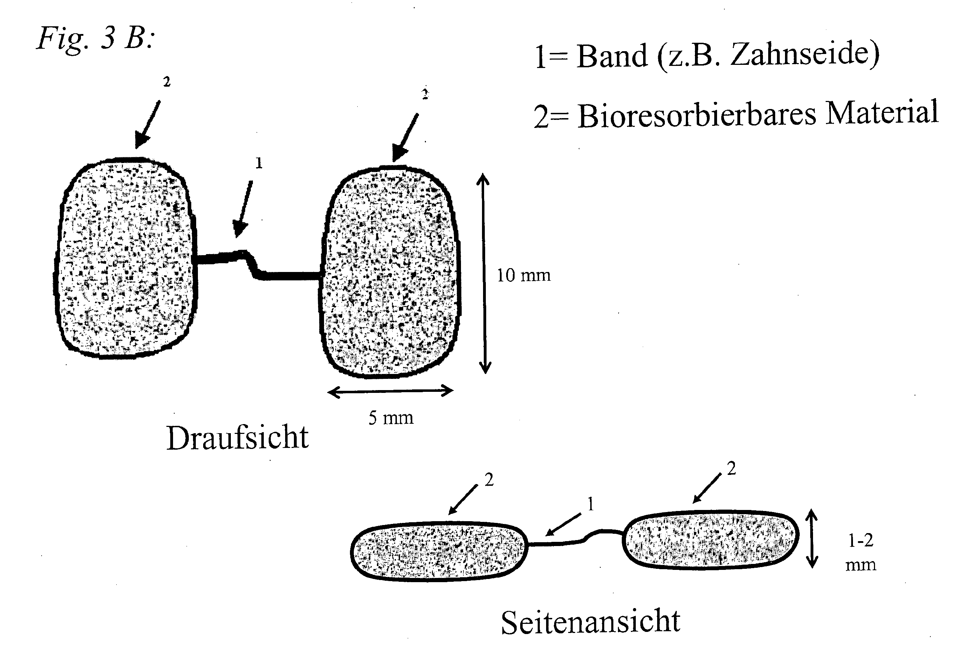

Das in Fig. 3 dargestellte Produkt zur Versorgung einzelner Zähne weist einen Kollagenschwamm mit einer Höhe von etwa 10 mm und einer Breite von etwa 5 mm auf, wobei die Dicke des Kollagenschwamms etwa 1 bis 2 mm beträgt. Wie besonders deutlich in Fig. 3A zu erkennen ist, sind an dem Kollagenschwamm Fixierungsbänder befestigt, die beispielsweise aus Zahnseide gebildet sein können. Gemäß Fig. 3B können auch 2 oder mehr Kollagenschwämme der in Fig. 3A dargestellten Art zur Versorgung unterschiedlicher Bereiche eines Zahns und/oder zur Versorgung nebeneinanderliegender Zähne mit Hilfe von beispielsweise aus Zahnseide gebildeten Fixierungsbändern miteinander verbunden sein.

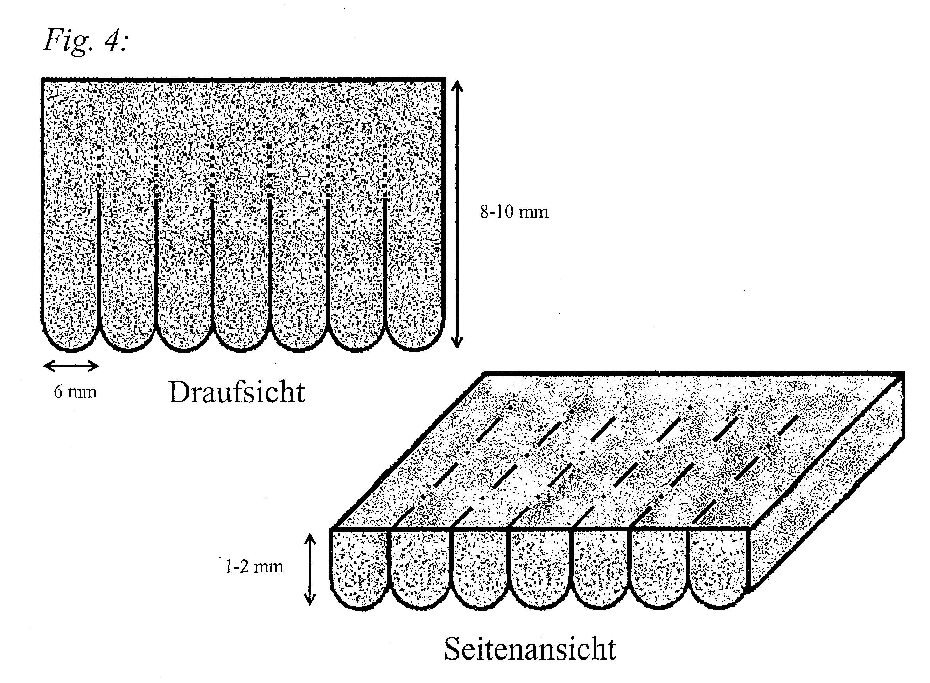

Bei dem in Fig. 4 dargestellten eingeschnittenen Kollagenschwamm zur Herstellung erfindungsgemäßer Produkte handelt es sich um einen Kollagenstreifen mit beliebiger Länge, einer Breite von etwa 8 bis 10 mm und einer Dicke von etwa 1 bis 2 mm. Wie besonders deutlich in der Seitenansicht zu erkennen ist, weist dieser Kollagenstreifen senkrecht zur Längsrichtung geführte Einschnitte auf, die einen Abstand von etwa 6 mm voneinander aufweisen. Der so vorbereitete Streifen kann beispielsweise zur Herstellung von in Fig. 3 dargestellten Produkten eingesetzt werden.

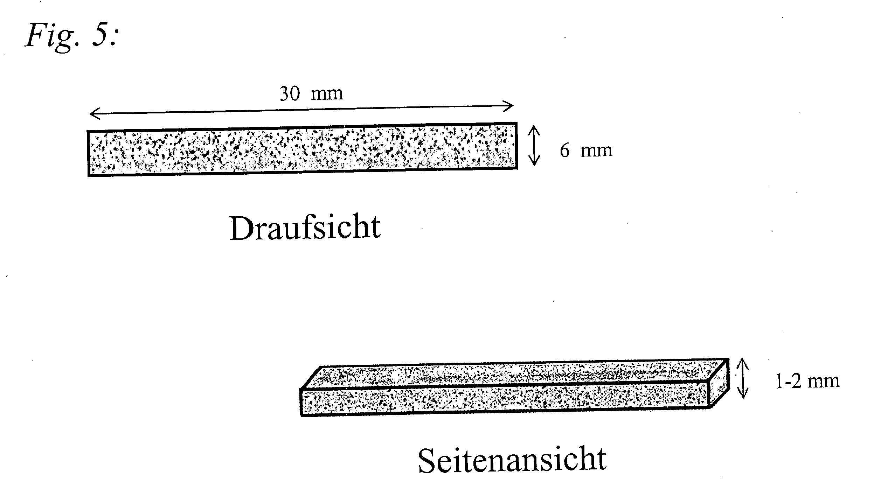

Der in Fig. 5 dargestellte Kollagenstreifen kann beispielsweise zur Herstellung von erfindungsgemäßen Produkten gemäß Fig. 2 eingesetzt werden.

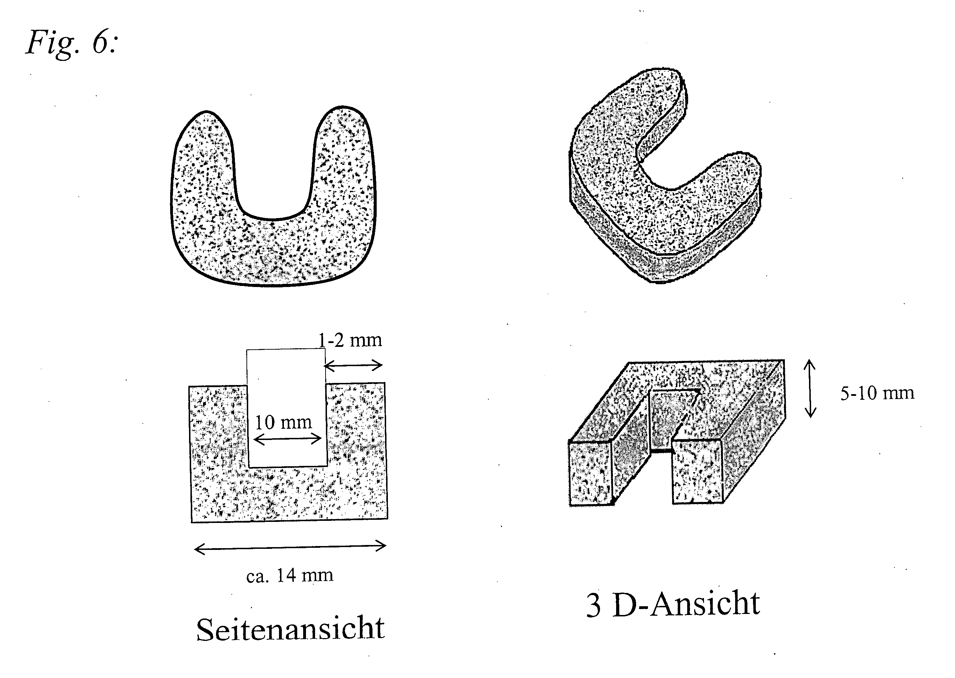

Zusätzlich oder alternativ kann der in Fig. 5 dargestellte Kollagenstreifen auch zur Herstellung von in Fig. 6 dargestellten Produkten eingesetzt werden.

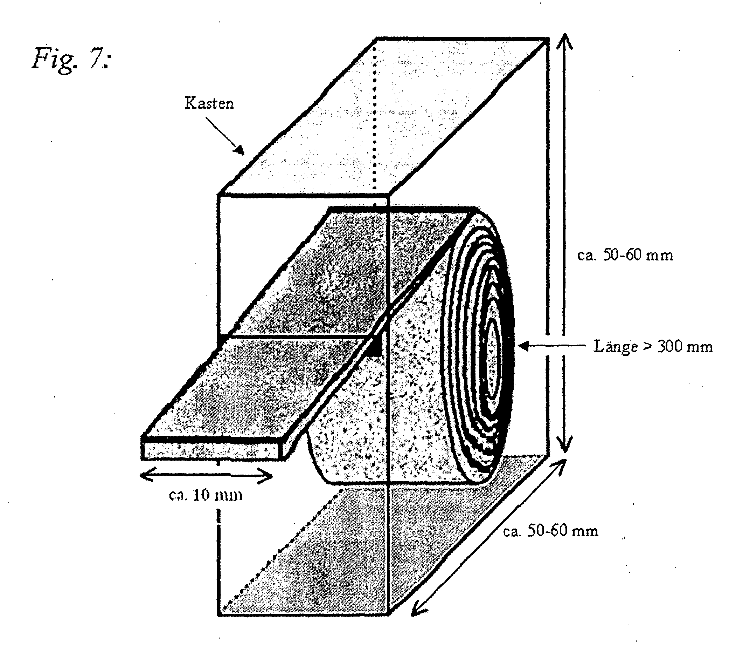

Der in Fig. 5 dargestellte Kollagenstreifen kann beispielsweise spiralförmig aufgewickelt und in einem Spender gemäß Fig. 7 untergebracht werden.

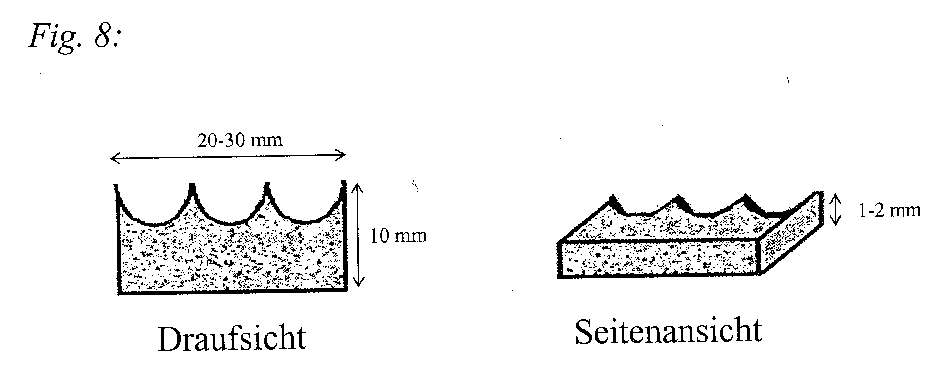

Bei dem in Fig. 8 dargestellten Produkt zur Versorgung des Zahnfleisches über einigen Zähnen ist eine Längskante des Streifens wellenförmig ausgeführt, um so eine Anpassung an die Zahneinschnitte im Zahnfleisch herbeizuführen.

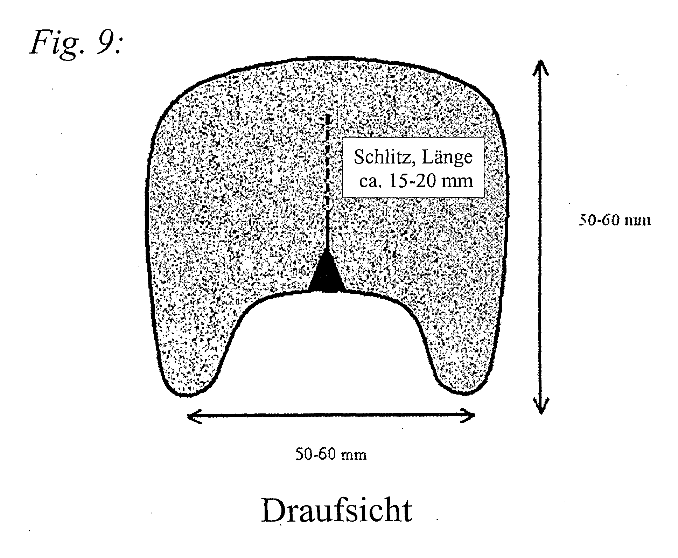

Bei dem in Fig. 9 dargestellten Produkt handelt es sich um eine Sonderform des in allgemeiner Form in Fig. 1B dargestellten Produktes, bei dem durch einen Einschnitt eine bessere Anpassung an die Prothese im Oberkiefer erreichbar ist.

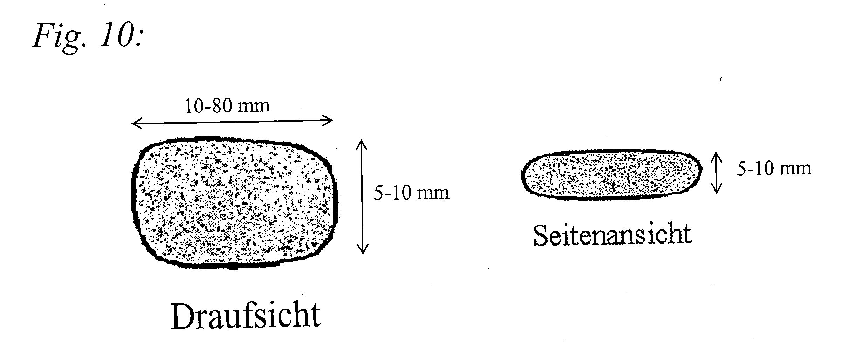

Das in Fig. 10 dargestellte Produkt ist im Mundraum universell einsetzbar.

Das in Fig. 11 dargestellte Produkt dient zur Erläuterung der vorstehend beschriebenen Kompressibilität erfindungsgemäßer Produkte.

Bei den vorstehend herausgestellten und in der Zeichnung angegebenen Bemessungsangaben handelt es sich um im Rahmen der Erfindung bevorzugte Bereiche. Es kann allerdings auch von diesen Bereichen abgewichen werden. Das gilt insbes. für die Anwendung erfindungsgemäßer Produkte bei Kindern. Bei dieser Anwendung können die in der Zeichnung dargestellten Formen erhalten bleiben, es werden jedoch die Abmessungen entsprechend verkleinert.

## Patentansprüche

1. Produkt zur Versorgung von Entzündungen, Druckstellen und/oder Aphthen im Oralbereich aus einem überwiegend, vorzugsweise vollständig, bioresorbierbaren Material, **dadurch gekennzeichnet, daß** das bioresorbierbare Material ein an der Schleimhaut haftendes, flexibles und/oder kompressibles Polsterelement aufweist, insbes. vollständig als Polsterelement ausgeführt ist.

2. Produkt nach Anspruch 1, **dadurch gekennzeichnet, daß** das bioresorbierbare Material ein vorzugsweise mit mindestens einem Begleitstoff versetztes Kollagen aufweist.

3. Produkt nach Anspruch 2, **dadurch gekennzeichnet, daß** das Kollagen bovines, equines, porkines und/oder marines Kollagen aufweist.

4. Produkt nach einem der vorhergehenden Ansprüche, **dadurch gekennzeichnet, daß** das bioresorbierbare Material Kollagen und mindestens ein weiteres bioresorbierbares Polymer aufweist.

5. Produkt nach Anspruch 4, **dadurch gekennzeichnet, daß** das bioresorbierbare Material als weiteres bioresorbierbares Polymer ein Alginat, Gellan, Chitosan, Chitin, κ-Karagenan, ϕ-Karagenan, Xanthan, Carboxymethylcellulose, Hydroxyethylcellulose und/oder Hydroxyethylpropylcellulose aufweist.

6. Produkt nach Anspruch 4 oder 5, **dadurch gekennzeichnet, daß** die Konzentration des weiteren bioresorbierbaren Materials 0,5 bis 50 %, bezogen auf das Trockengewicht des bioresorbierbaren Trägermaterials, beträgt.

7. Produkt nach einem der Ansprüche 2 bis 6, **dadurch gekennzeichnet, daß** das bioresorbierbare Material mindestens einen die Erhöhung der Haftung an der Schleimhaut, die Verbesserung der Flexibilität bzw. Anschmiegsamkeit und/oder die Verbesserung des Geschmacks bewirkenden Begleitstoff aufweist.

8. Produkt nach Anspruch 7, **dadurch gekennzeichnet, daß** der Begleitstoff Honig, Propolis, Aloe Vera, Panthenol, Polyethylenglykol 200-2000, Polyvinylpyrrolidon, Glycerin, Kamilleextrakt, Salbeiöl, Pfefferminzöl, Orangenextrakt, Zitronenextrakt, Cyclamat, aromatische Süßstoffe, Vitamine und/oder Provitamine aufweist.

9. Produkt nach einem der Ansprüche 7 oder 8, **dadurch gekennzeichnet, daß** der Begleitstoff in einer Konzentration von 0,5 bis 50 %, bezogen auf das Trockengewicht des Trägermaterials, vorliegt.

10. Produkt nach einem der Ansprüche 2 bis 9, **dadurch gekennzeichnet, daß** der Begleitstoff ein Anästhetikum und/oder Antiseptikum aufweist.

11. Produkt nach Anspruch 10, **dadurch gekennzeichnet, daß** das Antiseptikum bzw. Lokalanästhetikum aus der Gruppe der Ester bzw. Säureamide der Paraaminobenzoesäure ausgewählt ist.

12. Produkt nach Anspruch 10 oder 11, **dadurch gekennzeichnet, daß** dem Antiseptikum bzw. Lokalanästhetikum eine geringe Menge Adrenalin zur Wirkstoffverbesserung zugesetzt ist (Verdünnung: 1 : 200.000).

13. Produkt nach einem der Ansprüche 10 bis 12, **dadurch gekennzeichnet, daß** das Anästhetikum Ultracain, Lidocain, Mepivacain, Bupivacain, Prilocain, Articain, Procain und/oder Tetracain aufweist.

14. Produkt nach einem der Ansprüche 10 bis 13, **dadurch gekennzeichnet, daß** die Konzentration des Antiseptikums bzw. Lokalanästhetikums im Rahmen der physiologischen Wirksamkeit im Bereich von 0,1 bis 2 %, bezogen auf das Trockengewicht des Trägermaterials, liegt.

15. Produkt nach einem der Ansprüche 2 bis 14, **gekennzeichnet durch** mindestens einen eine antimikrobielle Verbindung aufweisenden Begleitstoff.

16. Produkt nach Anspruch 15, **dadurch gekennzeichnet, daß** die antimikrobielle Verbindung aus dem Bereich der Biguanide stammt.

17. Produkt nach Anspruch 16, **dadurch gekennzeichnet, daß** der Begleitstoff Polyhexamethylenbiguanid Hydrochlorid, Chlorhexidin Dihydrochlorid, Chlorhexidin Diacetat, Chlorhexidin D-Glukonat, Octenidin, Taurolidin, Cetylpyridiniumhydrochlorid und/oder Mischungen daraus aufweist.

18. Produkt nach einem der Ansprüche 15 bis 17, **dadurch gekennzeichnet, daß** die Konzentration der Begleitstoffe im Rahmen der antimikrobiellen Wirksamkeit im Bereich von 0,5 bis 2 %, bezogen auf das Trockengewicht des Trägermaterials, liegt.

19. Produkt nach einem der Ansprüche 15 bis 18, **dadurch gekennzeichnet, daß** die antimikrobielle Verbindung eine silberhaltige Verbindung aufweist.

20. Produkt nach Anspruch 19, **dadurch gekennzeichnet, daß** die silberhaltige Verbindung mindestens ein lösliches Silbersalz, schwerlösliches Silbersalz, Silberkomplexe, organische Silberverbindungen und/oder elementares Silber, vorzugsweise Silberchlorid, umfaßt.

21. Produkt nach Anspruch 19 oder 20, **dadurch gekennzeichnet, daß** die silberhaltigen Verbindungen Silbernitrat, Silberacetat, Silberkarbonat, Silberhalogenide, Silbersulfatiacin, Silberdiamin-Komplexe, Silberdithionat-Komplexe, Silberthiocyanat, Silberpulver und/oder Mischungen daraus aufweisen.

22. Produkt nach einem der Ansprüche 18 bis 21, **dadurch gekennzeichnet, daß** die Konzentration der Begleitstoffe im Rahmen der antimikrobiellen Wirksamkeit im Bereich von 0,1 bis 2 %, bezogen auf das Trockengewicht des Trägermaterials, liegt.

23. Produkt nach einem der vorhergehenden Ansprüche, **dadurch gekennzeichnet, daß** das bioresorbierbare Material einen feinporigen Schwamm aufweist.

24. Produkt nach einem der vorhergehenden Ansprüche, **dadurch gekennzeichnet, daß** das bioresorbierbare Material einen Schaum bzw. Schwamm aufweist, der in Z-Richtung (vgl. Fig. 11) eine Dicke von 0,5 bis 4 mm aufweist.

25. Produkt nach einem der vorhergehenden Ansprüche, **dadurch gekennzeichnet, daß** es durch Strahlung (25 kGy) oder durch Begasen mit Ethylenoxid sterilisierbar ist.

26. Produkt nach einem der vorhergehenden Ansprüche, **dadurch gekennzeichnet, daß** sich das bioresorbierbare Material durch eine kurze Druckeinwirkung von einem Bar in Z-Richtung (vgl. Fig. 11) auf weniger als 50 %, vorzugsweise weniger als 40 %, insbes. 25 % oder weniger, der ursprünglichen Dicke komprimieren läßt.

27. Produkt nach einem der vorhergehenden Ansprüche, **dadurch gekennzeichnet, daß** das bioresorbierbare Material, vorzugsweise bedingt durch einen Lyophilisationsprozeß, eine gute Verformbarkeit in X- und Y-Richtung (vgl. Fig. 11) aufweist.

28. Produkt nach einem der vorhergehenden Ansprüche, **dadurch gekennzeichnet, daß** das bioresorbierbare Material im Dentalbereich abbaubar ist und bei einem versehentlichen Verschlucken vollständig resorbiert wird.

29. Produkt nach einem der vorhergehenden Ansprüche, **dadurch gekennzeichnet, daß** das bioresorbierbare Material zur Verringerung der Biodegradierbarkeit zumindest teilweise vernetzt ist.

30. Produkt nach einem der vorhergehenden Ansprüche, **dadurch gekennzeichnet, daß** das bioresorbierbare Material mit bi- bzw. multifunktionellen Aldehyden, Carbonsäuren, Carbonsäurechloriden, Carbonsäurealdehyden und/oder Epoxiden vernetzbar ist.

31. Produkt nach einem der vorhergehenden Ansprüche, **dadurch gekennzeichnet, daß** das bioresorbierbare Material mit spaltbaren Brücken oder durch γ-Bestrahlung oder durch β-Bestrahlung hinsichtlich der Biodegradierbarkeit veränderbar ist.

32. Produkt nach Anspruch 31, **dadurch gekennzeichnet, daß** als spaltbare Vernetzungsbrücken Hydroxyethyl methacrylate einsetzbar sind.

33. Produkt nach einem der vorhergehenden Ansprüche, **dadurch gekennzeichnet, daß** das bioresorbierbare Material zur Wundheilung und/oder zur Desinfektion (z.B. Behandlung von Aphthen, Druckstellen, Mykosen, Soor und anderen Infektionen) einsetzbar ist.

34. Produkt nach einem der vorhergehenden Ansprüche, **dadurch gekennzeichnet, daß** das bioresorbierbare Material als Polster zwischen Zahnersatz bzw. Zahnspangen und der Mundschleimhaut einer Wunde oder dem Zahnfleisch einsetzbar ist.

35. Produkt nach Anspruch 1, **dadurch gekennzeichnet, daß** das bioresorbierbare Material als Haftmittel für Voll- oder Teilprothesen einsetzbar ist.

36. Produkt nach einem der vorhergehenden Ansprüche, **dadurch gekennzeichnet, daß** es bspw. durch Chitosanzusatz zur Hämostase bei Blutungen im Dentalbereich, auch bei Personen, die unter dem Einfluß von Blutgerinnungshemmern, wie z.B. Marcumar, stehen, einsetzbar ist.

37. Produkt nach einem der vorhergehenden Ansprüche, **gekennzeichnet durch** ein Haftungsvermögen von 1 Newton oder mehr, insbes. 2 Newton oder mehr, besonders bevorzugt 4 Newton oder mehr.

38. Verwendung eines Produktes nach einem der vorhergehenden Ansprüche, zur Behandlung von Entzündungen, Druckstellen oder Aphthen im Oralbereich.

39. Verwendung nach Anspruch 38, **dadurch gekennzeichnet, daß** das bioresorbierbare Material als Polster zwischen Zahnersatz bzw. Zahnspangen und der Mundschleimhaut, einer Wunde und/oder dem Zahnfleisch eingesetzt wird.

40. Verwendung nach Anspruch 38 oder 39, **dadurch gekennzeichnet, daß** das bioresorbierbare Material als Haftmittel für Voll- oder Teilprothesen eingesetzt wird.

41. Verwendung nach einem der Ansprüche 38 bis 40, **dadurch gekennzeichnet, daß** das bioresorbierbare Material zur Hämostase bei Blutungen im Oralbereich eingesetzt wird.
